Europäisches Patentamt

European Patent Office

Office européen des brevets

(11) Publication number: **0 225 948 B1**

# EUROPEAN PATENT SPECIFICATION

(45) Date of publication of patent specification: **20.03.91**

(51) Int. Cl.5: **A61K 9/28**, A61K 9/30, C09B 67/00

(21) Application number: **85308938.1**

(22) Date of filing: **09.12.85**

(54) **Dry pigment system.**

(43) Date of publication of application:
**24.06.87 Bulletin 87/26**

(45) Publication of the grant of the patent:
**20.03.91 Bulletin 91/12**

(84) Designated Contracting States:
**AT BE CH DE FR GB IT LI LU NL**

(56) References cited:
**EP-A- 0 101 087**      **CA-A- 935 255**
**DE-A- 2 535 447**     **DE-B- 1 147 192**
**GB-A- 2 065 691**     **US-A- 2 613 160**

**Concise Chemical and Technical Dictionary**

(73) Proprietor: **CROMPTON & KNOWLES CORPORATION**
**One Station Place Metro Center**
**Stamford, Connecticut 06902(US)**

(72) Inventor: **Heinze, Richard**
**284 White Oak Ridge Road, Bridgewater**
**New Jersey(US)**

(74) Representative: **Hardisty, David Robert et al**
**BOULT, WADE & TENNANT 27 Furnival Street**
**London EC4A IPQ(GB)**

## Description

The present invention relates to the art of color coating such edible consumer products as pharmaceutical or veterinary tablets and confectionary pieces. More particularly, the invention is directed to a dry pigment composition used for making a color coating suspension. However, the invention also has application to the internal coloring of products, for example, the internal coloring of confectionary.

The color coating of products can be advantageously accomplished by applying a film forming pigment containing suspension, which typically includes the presence of a film forming polymer. In other cases, the color coating of products is appropriately accomplished by applying a sugar syrup suspension containing an aqueous dispersion of pigment.

The general technique of film coating and sugar coating are known in the prior art. For example, U.S. Patent No. 2,954,323 to Endicott et. al. discloses the improved efficiency of the film coating process and the superior coating properties of the products made thereby.

The coating suspensions used in film coating or sugar coating are typically made from commercially available pigment suspensions, which are conventionally concentrated non-aqueous dispersions of lake pigments, usually including a colloid such as polyvinylpyrollidone for maintaining the pigment in dispersion. The pigment dispersions are typically stirred into a larger volume of polymer solution or sugar solution to form the color coating suspensions. Examples of such pigment dispersions and their use are described in U.S Patent No. 3,981,984 to Signorino.

The purpose of the present invention was to obtain a dry composition, rather than a liquid pigment suspension, which could be added directly to a polymer solution or sugar syrup solution to form a color coating suspension. Dry compositions have heretofore not been widely used because of the difficulties involved with their application. Dry compositions have a tendency to agglomerate and resist dispersion in solution. This can result in a poor quality coating, since the coating, in the form of a very thin film, should be uniform and consistent from one batch of tablets to the next. It is desired to obtain a coating exhibiting a smooth, polished, and elegant appearance which requires well dispersed pigment particles. However, this has been difficult to obtain with a dry composition, and the results have been unsatisfactory.

U.K. Patent Application GB 2065691A, by Porter et al, published on July 1, 1981, discloses a method of making a dry film coating composition which comprises the steps of mixing a polymer powder, such as methylcellulose, and pigment particles in a blender, adding a plasticizer such as polyethylene glycol to the blender containing the polymer-pigment mix, and mixing until the combined mix is thoroughly blended. A surfactant or a flow aid is optional.

We draw attention to our co-pending applications Nos. 85308937.3 (Publication No. EP-A-0228485) and 85308939.9 (Publication No. EP-A-0226671).

The present applicant has produced a dry composition for use in film coating or sugar coating, which comprises pigment particles and a small quantity of what is referred to as a dispersing agent, which has an extraordinary effect on the pigment particles.

Accordingly the present invention relates to the use of a dry pigment composition comprising a dry mixture of a pigment and a dispersing agent, wherein the pigment is an edible lake pigment and the dispersing agent is a salt of an organic compound containing at least one carboxy group or a salt of phosphoric acid or a mixture of the salt with the acid form thereof, wherein the dispersing agent is present at 0.005 to 5% by weight of the combined weight of the pigment and dispersing agent, in the coating or colouring of pharmaceutical or veterinary tablets or confectionary pieces or other foodstuff.

The dispersing agent affects the pigment particles in the dry state as well as facilitating dispersion into solution so that the dry composition may form uniform small particles that maintain a striking degree of separation. The particles do not agglomerate as hitherto, and as a result, a more uniform coating is obtained. The solubility of the composition in the film forming solution may increase by a factor of five or more. The phenomenon known as specking, caused by larger sized pigment particles that do not disperse, is rendered insignificant or absent.

The present composition need not contain the high levels of polymer or plasticizer required in U.K. Patent Application GB 2065671A, referred to above. The present composition is useful in both aqueous and organic film coating and in sugar coating as well. The composition readily disperses in liquid as a result of the dispersing agent.

The advantage of a dry pigment composition in comparison to a liquid pigment suspension is great. The dry pigment composition would have virtually unlimited stability and storage life. There is no danger of the pigment particles settling out and hardening, as is liable to occur with pigment suspensions. The necessity for preservatives and the danger of microcontamination is reduced or avoided. Since the composition weighs considerably less than the liquid composition, the expense of shipment and the cost of the product is reduced. The presence of inflammable or deleterious organic solvents is obviated.

The present invention is concerned with the provision of a dry pigment composition for use in a film forming solution or sugar solution, or for use for the internal coloring of confectionary such as jelly beans. It is desired that the composition readily disperses in solution.

The present invention is further described in the following detailed description of preferred embodiments of the invention.

The pigments suitable for use in the context of the present invention include pigment hydroxide lakes, which are dyes combined with a metal hydrate substratum. A variety of lakes, including lakes incorporating azo, triphenylmethane, fluorescein, and sulfonated indigo dyes, are suitable in the present invention. Natural lakes such as carmine are suitable. FD&C lakes are suitable for application in food, drug, and cosmetic products. D&C lakes such as D&C Red 27 are suitable for pharmaceutical and cosmetic application.

Lakes have been developed with a wide range of strengths. For food and confectionary applications, the mid-range dye content lakes are the most useful. These lakes are manufactured by Crompton & Knowles Corporation of Fairlawn, New Jersey (hereinafter referred to as "C&K"), and Warner-Jenkinson Manufacturing Company of Saint Louis, Missouri (hereinafter referred to "W-J"). For example, the following pigments are commercially available from C&K or W-J:

| Yellow | # 6/40% |
|--------|---------|
| Yellow | # 5/36% |
| Blue   | # 1/11% |
| Blue   | # 2/39% |
| Red    | #40/40% |
| Red    | #27/36% |
| Red    | # 3/40% |

A 50/50 combination of C&K and W-J lakes may be preferrable. In general, it has been found that W-J lakes tend to have a higher tint, but may cause thickening. By using a mixture of W-J and C&K lakes, both a high tint and non-thickening is more readily obtained. Of course, developments and changes in the lakes by their manufacturers may require a reassessment, as would be understood by those skilled in the art, of the properties of a particular brand in regard to use in the present invention.

The dispersing agent is most effectively a salt of an organic, carboxyl containing compound or a mixture thereof with the acid form. It may also be a salt of phosphoric acid optionally together with phosphoric acid. Preferred dispersing agents include salts of compounds having one to four carboxylic groups. Dispersing agents include, but are not limited to salts of adipic acid, benzoic acid, citric acid, fumaric acid, succinic acid, maleic acid, lactic acid, tartaric acid, ascorbic acid, and propionic acid and mixtures with the acid thereof. Trisodium phosphate has been found to work in some cases as a dispersing agent, but in general not as well as the above mentioned organic, carboxylic containing compounds.

In general, the dispersing agents must, at least partly, be in the salt form. A 50/50 combination of the salt and acid is suitable. For example, sodium citrate, by itself, or a mixture of sodium citrate and citric acid, produces excellent results. It is surmised that the dispersing agent complexes or is absorbed by the lake pigment particles. Due to the wide variety of dyes present in lake pigments, it is further surmised that the metal hydroxide substrate of the FD&C lakes may function in the complexing of the dispersing agent. The resulting complex is believed to have electronic properties such that they repell another such complex, thereby resulting in readily and uniformly dispersed pigment particles.

The presence of one of the dispersing agents results in a dramatic change in the properties exhibited by the pigment composition, both in the dry form and in solution. The composition may readily disperse in solution and neither agglomerate nor clump. The dispersing agent is preferably present in an amount of 0.005 to 5 percent by weight in the mixture. The agent is more preferably present in an amount of 0.02 to 3 percent and most preferably, in an amount of 0.1 to 1.5 percent. In many cases, even a relatively very small amount of dispersing agent can drastically and favorably affect the properties of the composition.

The following Examples are intended to illustrate the invention.

Example 1

In a blender, the following components were weighed out and mixed.

| Component | Weight |
|---|---|
| Titanium dioxide 3328 | 49.99 g |
| Red 40/40 | 24.99 g |
| Yellow 6/40 | 24.99 g |
| Sodium Citrate | 1.50 g |

This composition readily dispersed in a sugar solution composed of a mixture of water and sucrose in a ratio of 3 to 7. This dispersion exhibited excellent coating properties.

Example 2

A drawdown is a test used to simulate the coating process. A few drops of suspension to be tested are placed in a line across a glass plate. Then a metal arm which is spaced a fraction of a millimeter from the glass plate is used to draw the suspension across the plate so as to produce a thin film. By examining this thin film, non-uniformity or other possible defects in the suspension can be observed. If the drawdown is clear and uniform, it is expected that the suspension will produce a good coating.

In a blender, the following components were weighed out and mixed:

| Component | Weight |
|---|---|
| Blue 2/39 | 50 g |
| Titanium dioxide 3328 | 50 g |
| Sodium Citrate | 0.5 g |

This composition was dispersed in a sugar syrup solution, Drawdowns of the dispersion were made to determine the degree of dispersion and the unformity of the dispersion. The results were excellent.

Comparative Example 3

The composition of Example 1 was repeated, except that sodium citrate was not included. The composition was as follows:

| Component | Weight |
|---|---|
| Titanium dioxide 3328 | 50 g |
| Red 40/40 | 25 g |
| Yellow 6/40 | 25 g |

The composition of Example 3 was compared to the composition of Example 1. It is noted that the lakes of Examples 1 and 3 were obtained from the same lot so that the effect of the presence of the dispersing agent could be more accurately evaluated. The comparison involved side-by-side drawdowns and microscopic comparison of the solutions. The composition of Example 1 was found to be significantly superior to the composition of Example 3 in every respect. The dry blend with sodium citrate exhibited uniformly small particles, whereas the dry blend without sodium citrate exhibited agglomerates. Moreover, the particles present in the composition of comparative example were clearly not as uniform and relatively larger in average diameter.

With respect to dispersing, the effect of the addition of sodium citrate was also noticeable. The dry blend with sodium citrate readily dispersed in about 1 to 2 minutes. In contrast, the dry blend without sodium citrate required at least ten minutes to mix into the solution.

Example 4

The following components were weighed out and blended:

| Component | Weight |
|---|---|
| Titanium dioxide 3328 | 20 g |
| Yellow 6/39 | 20 g |
| Sodium Citrate | 2 g |

This composition of the present invention was compared to the composition of comparative Example 5 below.

Comparative Example 5

The dry blend of the present invention as illustrated by Example 4 was compared to the use of a dry blend not containing sodium citrate. The lakes in both examples were taken from the same lot. This comparative example consisted of the following:

| Component | Weight |
|---|---|
| Titantium dioxide | 20 g |
| Yellow 6/39 | 20 g |

The compositions of Example 4 and Comparative Example 5, respectively, were added to a 70/30 sugar syrup solution at a weight concentration of 1.50 percent. Drawdowns and visual microscopic observation were made of the respective dispersions.

It was found that the dispersion made with the composition of Example 4 exhibited dispersed particles that were significantly smaller and finer in size. The dry blend without sodium citrate required at least five times as long to disperse in the sugar syrup solution.

Comparative Example 6

The following components were weighed out and mixed:

| Component | Weight |
|---|---|
| Titanium dioxide 3328 | 15.00 g |
| Yellow 6/39 | 15.00 g |
| Polyvinylpyrollidone | 1.00 g |
| Sugar | 41.40 g |
| Distilled water | 27.60 g |

A quantity of this mixture was added to a 70/30 sugar water solution. The suspension was mixed well for one half hour using a magnetic stirrer and heating to 80° C. Drawdowns and microscopic observations were made of the mixture.

This dispersion was found to be inferior to the composition of the present invention exemplified by Example 4 above. In comparison, the dispersion of Example 4 showed particles of significantly smaller and finer size.

The composition of the present invention may contain additional additives. For example, in order to make the composition dustless, a non-dusting additive such as propylene glycol, glycerine, or dioctyl sodium sulfosuccinate (DSS) may be included in an effective amount of about five percent.

It is to be understood that the foregoing detailed description and preferred embodiments are merely given by way of illustration, and that modifications may be made, within the skill of the art, and the scope of the invention.

**Claims**

1. The use of a dry pigment composition comprising a dry mixture of a pigment and a dispersing agent, wherein the pigment is an edible lake pigment and the dispersing agent is a salt of an organic compound containing at least one carboxy group or a salt of phosphoric acid or a mixture of the salt with the acid form thereof, wherein the dispersing agent is present at 0.005 to 5% by weight of the combined weight of the pigment and dispersing agent, in the coating or colouring of pharmaceutical or veterinary tablets or confectionary pieces or other foodstuff.

2. The use as claimed in claim 1 wherein the dispersing agent is present at 0.1 to 0.15% by weight.

3. The use as claimed in claim 1 or claim 2 wherein the pigment comprises an FD&C lake or a D&C lake or carmine.

4. The use as claimed in any one of claims 1 to 3 wherein the pigment further comprises titanium dioxide.

5. The use as claimed in any one of the preceding claims wherein the dispersing agent is a salt of tartaric acid, citric acid, fumaric acid, adipic acid, maleic acid or a mixture of the salt with the acid form thereof, or trisodium phosphate.

6. The use as claimed in claim 1 of a dry composition comprising a dry mixture of pigment selected from an FD&C lake, a D&C lake or carmine and additionally 0.005 to 5%, at least partly in the salt form of an organic compound containing one to four carboxylic acid groups, or phosphoric acid; or a composition comprising a pigment and 0.1 to 0.5% of a salt of an organic compound containing at least one carboxylic acid group or a salt of phosphoric acid.

7. The use as claimed in any one of the preceding claims wherein the composition further comprises propylene glycol, glycerine, or dioctyl sodium sulfosuccinate or another non-dusting agent or sugar.

8. The use as claimed in any one of the preceding claims wherein the composition is admixed with a suitable liquid medium.

9. The use as claimed in claim 8, wherein the liquid medium is a film forming solution or a sugar solution.

**Revendications**

1. Emploi d'une composition de pigment sec comprenant un mélange sec d'un pigment et d'un agent dispersant, dans lequel le pigment est un pigment laqué comestible et l'agent dispersant est un sel d'un composé organique contenant au moins un grcupement carboxyle ou un sel d'acide phosphorique ou un mélange du sel avec sa forme acide, dans lequel l'agent dispersant est présent à raison de 0,005 à 5 % en poids des poids réunis du pigment et de l'agent dispersant, pour l'enrobage ou la coloration de comprimés pharmaceutiques ou vétérinaires ou de friandises ou d'autres denrées alimentaires.

2. Emploi selon la revendication 1, dans lequel l'agent dispersant est présent à raison de 0,1 à 0,15 % en poids.

3. Emploi selon la revendication 1 ou la revendication 2, dans lequel le pigment comprend une laque FD & C ou une laque D & C ou du carmin.

4. Emploi selon l'une quelconque des revendications 1 à 3, dans lequel le pigment comprend en outre du dioxyde de titane.

5. Emploi selon l'une quelconque des revendications précédentes, dans lequel l'agent dispersant est un sel d'acide tartrique, d'acide citrique, d'acide fumarique, d'acide adipique, d'acide maléique ou un mélange du sel avec sa forme acide, ou du phosphate trisodique.

6. Emploi selon la revendication 1 d'une composition sèche comprenant un mélange sec de pigment sélectionné parmi une laque FD & C, une laque D & C ou du carmin et additionnellement 0,005 à 5 % d'un composé organique se présentant au moins partiellement sous forme de sel et contenant 1 à 4 groupements acide carboxylique ou de l'acide phosphorique ; ou une composition comprenant un pigment et 0,1 à 0,5 % d'un sel d'un composé organique contenant au moins un groupement acide carboxylique ou un sel d'acide phosphorique.

7. Emploi selon l'une quelconque des revendications précédentes, dans lequel la composition comprend en outre du propylèneglycol, du glycérol ou du dioctylsulfosuccinate de sodium ou un autre agent non formateur de poussière ou du sucre.

8. Emploi selon l'une quelconque des revendications pré-cédentes, dans lequel la composition est mélangée à un milieu liquide approprié.

9. Emploi selon la revendication 8, dans lequel le milieu liquide est une solution filmogène ou une solution de sucre.

**Ansprüche**

1. Verwendung einer trockenen Pigmentzusammensetzung, die eine Trockenmischung eines Pigments und eines Dispergiermittels ist, wobei das Pigment ein für den Verzehr geeignetes Lackpigment und das Dispergiermittel ein Salz einer organischen Verbindung mit mindestens einer Carboxy-Gruppe oder ein Salz der Phosphorsäure oder eine Mischung des Salzes mit dessen Säureform ist, und wobei das Dispiergiermittel in einer Menge von 0,005 bis 5 Gew-% des Gesamtgewichtes von Pigment und Dispergiernittel vorliegt, zum Überziehen oder Färben von pharmazeutischen oder tiermedizinischen Tabletten oder Süßwarenstücken oder anderen Lebensmitteln.

2. Verwendung nach Anspruch 1, dadurch **gekennzeichnet,** daß das Dispergiermittel mit 0,1 bis 0,15 Gew-% enthalten ist.

3. Verwendung nach Anspruch 1 oder 2, dadurch **gekennzeichnet,** daß das Pigment ein FD&C- oder ein D&C-Lackpigment oder Karmin enthält.

4. Verwendung nach jedem der Ansprüche 1 bis 3, dadurch **gekennzeichnet,** daß das Pigment weiterhin Titandioxyd enthält.

5. Verwendung nach jedem der vorstehenden Ansprüche, dadurch **gekennzeichnet,** daß das Dispergiernittel ein Salz der Weinsäure, Zitronensäure, Fumarsäure, Adipinsäure, Maleinsäure oder eine Mischung aus Salzen mit deren Säureform oder Trinatriumphosphat ist.

6. Verwendung nach Anspruch 1, dadurch **gekennzeichnet,** daß die trockene Zusammensetzung aus einer trockenen Mischung aus Pigmenten, ausgewählt aus FD&C-, D&C-Lackpigmenten oder Karmin und zusätzlich 0,005 bis 5 % einer organischen Verbindung besteht, die wenigstens teilweise in Salzform vorliegt und 1 bis 4 Carboxyl-Säuregruppen oder Phosphorsäure enthält, oder aus einer Zusammensetzung besteht, die ein Pigment und 0,1 bis 0,5 % eines Salzes einer organischen Verbindung, die wenigstens eine Carboxylsäuregruppe oder ein Salz der Phosphorsäure enthält.

7. Verwendung nach jedem der vorstehenden Ansprüche, dadurch **gekennzeichnet,** daß die Zusammensetzung weiterhin Propylenglykol, Glyzerin oder Hexadekan-Natrium-Sulfosuccinat oder ein anderes, nicht staubendes Mittel oder Zukker enthält.

8. Verwendung nach jedem der vorstehenden Ansprüche, dadurch **gekennzeichnet,** daß die Zusammensetzung mit einem geeigneten flüssigen Medium vermischt ist.

9. Verwendung nach Anspruch 8, dadurch **gekennzeichnet,** daß das flüssige Medium eine filmbildende Lösung oder eine Zuckerlösung ist.